(19) Europäisches Patentamt
European Patent Office
Office européen des brevets

(11) Veröffentlichungsnummer: **0 054 002**
**B1**

(12) # EUROPÄISCHE PATENTSCHRIFT

(45) Veröffentlichungstag der Patentschrift: **21.08.85**

(51) Int. Cl.⁴: **C 07 D 231/08**

(21) Anmeldenummer: **81810476.2**

(22) Anmeldetag: **04.12.81**

(54) **Verfahren zur Herstellung von 3-Pyrazolidinonen und ihre Verwendung in photographischen Entwicklerlösungen.**

(30) Priorität: **10.12.80 CH 9100/80**
**12.06.81 CH 3879/81**
**14.08.81 CH 5262/81**

(43) Veröffentlichungstag der Anmeldung: **16.06.82 Patentblatt 82/24**

(45) Bekanntmachung des Hinweises auf die Patenterteilung: **21.08.85 Patentblatt 85/34**

(84) Benannte Vertragsstaaten: **BE CH DE FR GB IT LI NL**

(56) Entgegenhaltungen:
**EP - A - 0 009 837**
**FR - A - 1 454 177**
**GB - A - 1 093 281**

(73) Patentinhaber: **CIBA-GEIGY AG, Postfach, CH-4002 Basel (CH)**

(72) Erfinder: **Fryberg, Mario, Dr., Dery-le-Mont, CH-1724 Praroman-le-Mouret (CH)**
Erfinder: **Stauner, Thomas, Dr., Route de Montiver 14, CH-1723 Marly (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 002 B1

BUNDESDRUCKEREI BERLIN

**Beschreibung**

Die vorliegende Erfindung betrifft ein Verfahren zur Herstellung von 3-Pyrazolidinonen.

Ein Verfahren zur Herstellung von 1-Phenyl-4,4-dihydroxymethyl-pyrazolidin-3-on, 1-p-Tolyl-4,4-dihydroxymethyl-pyrazolidin-3-on und 1-Phenyl-4-methyl-4-hydroxymethyl-pyrazolidin-3-on ist bereits aus GB 1 157 617 bekannt. Danach wird ein β-Hydroxycarbonsäureester mit einem Arylhydrazin in Gegenwart eines Alkoholats zum entsprechenden Arylhydrazid umgesetzt und anschließend mit z. B. wasserfreier p-Toluolsulfonsäure unter Erwärmen zum 3-Pyrazolidinon zyklisiert. 3-Pyrazolidinone können nach EP 9 837 auch nach einem Zweistufenprozeß hergestellt werden worin eine Hydrazin- und Carbonsäurekomponente zu einem Hydrazid reagieren, das in einem zweiten Reaktionsschritt zum entsprechenden 3-Pyrazolidinon zyklisiert wird.

Diese Verfahren vermögen noch nicht in allen Punkten zu befriedigen, da sie über mehrere Stufen geführt werden müssen und relativ lange Reaktionszeiten benötigen.

Aufgabe der vorliegenden Erfindung ist es daher, ein Verfahren zur Herstellung von 3-Pyrazolidinonen, bereitzustellen, welches über weniger Zwischenstufen, in kürzeren Reaktionszeiten und in höheren Ausbeuten zum gewünschten Produkt führt.

Es wurde nun gefunden, daß die erfindungsgemäße Aufgabe durch direkte Bildung des Hydrazids aus Carbonsäure- und Hydrazinkomponente und anschließender Zyklisierung in einstufiger Reaktion gelöst werden kann.

Zur Bildung sowie zur Zyklisierung des Hydrazids ist die Anwesenheit eines sauren Katalysators erforderlich.

Gegenstand der vorliegenden Erfindung ist somit ein Verfahren zur Herstellung von 3-Pyrazolidinonen der Formel

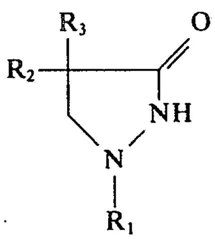

(1)

worin $R_1$ Phenyl oder durch Alkyl oder Alkoxy mit je 1 bis 4 Kohlenstoffatomen, Hydroxyl oder Halogen substituiertes Phenyl, $R_2$ Alkyl oder Hydroxyalkyl mit je 1 bis 4 Kohlenstoffatomen und $R_3$ Hydroxyalkyl mit 1 bis 4 Kohlenstoffatomen ist, durch Umsetzung einer β-Hydroxypropionsäure der Formel

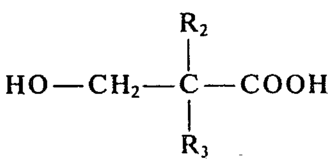

(2)

worin $R_2$ und $R_3$ die oben angegebenen Bedeutungen haben, mit einem Hydrazin der Formel

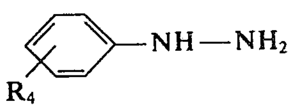

(3)

worin $R_4$ Wasserstoff, Alkyl oder Alkoxy mit je 1 bis 4 Kohlenstoffatomen, Hydroxyl oder Halogen ist, zu einem β-Hydroxypropionsäurehydrazid und Zyklisierung dieses Hydrazids in Gegenwart einer Säure, dadurch gekennzeichnet, daß man das β-Hydroxypropionsäurehydrazid in der gleichen Reaktionsmischung zyklisiert, wobei die Bildung des Hydrazids und dessen Zyklisierung in Gegenwart eines sauren Katalysators durchgeführt werden.

Gegenstand der vorliegenden Erfindung ist ferner ein Verfahren zur Herstellung von 3-Pyrazolidinonen, welches dadurch gekennzeichnet ist, daß Bildung und Zyklisierung des aus den Verbindungen der Formel (2) und (3) erhaltenen Hydrazids in der gleichen Schmelze erfolgen, wobei ein saurer Katalysator bei beiden Reaktionsschritten, oder auch nur bei der Zyklisierung anwesend ist.

Gegenstand der vorliegenden Erfindung ist auch ein Verfahren zur Herstellung von 3-Pyrazolidinonen, wonach nur die Zyklisierung des Hydrazids in der Schmelze in Gegenwart eines sauren Katalysators erfolgt. Der Katalysator kann in diesem Verfahren aber auch schon während der Hydrazidbildung im Reaktionsgemisch vorhanden sein.

Der Substituent $R_1$ in Formel (1) bedeutet gegebenenfalls substituiertes Phenyl der Formel

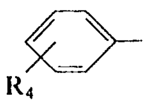

Darin bedeutet, $R_4$ Wasserstoff, Alkyl oder Alkoxy mit je 1 bis 4 Kohlenstoffatomen, wie z. B. Methyl, Propyl, i-Propyl oder Butyl sowie Methoxy oder Butoxy. Bevorzugt sind Methyl und Methoxy. $R_4$ bedeutet ferner Hydroxyl oder Halogen. Bevorzugtes Halogen ist Chlor. Die bevorzugte Bedeutung von $R_1$ ist Phenyl.

$R_2$ ist Alkyl oder Hydroxyalkyl mit je 1 bis 4 Kohlenstoffatomen, wie z. B. Methyl, Aethyl, Hydroxymethyl und 1- oder 2 Hydroxyäthyl. Besonders geeignet sind Methyl und Hydroxymethyl.

$R_3$ bedeutet Hydroxyalkyl mit 1 bis 4 Kohlenstoffatomen. Beispiele sind Hydroxymethyl, 1- oder 2-Hydroxyäthyl und 1- oder 4-Hydroxybutyl. Vorzugsweise bedeutet $R_3$ Hydroxymethyl.

Vorzugsweise setzt man im erfindungsgemäßen Verfahren eine $\beta$-Hydroxypropionsäure der Formel

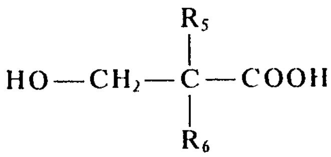 (4)

worin $R_5$ Methyl, Aethyl, Hydroxymethyl, 1- oder 2-Hydroxyäthyl und $R_6$ Hydroxymethyl oder 1- oder 2-Hydroxyäthyl ist, insbesondere der Formel

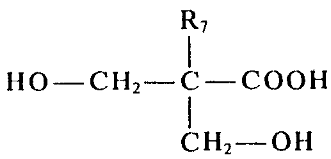 (5)

worin $R_7$ Methyl oder Hydroxymethyl ist, mit einem Phenylhydrazin der Formel

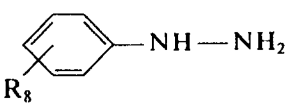 um, (6)

worin $R_8$ Wasserstoff, Methyl, Methoxy, Hydroxyl oder Chlor ist.

Für das erfindungsgemäße Verfahren besonders geeignete Phenylhydrazine entsprechen der Formel

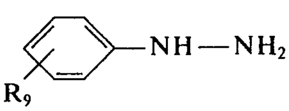 (7)

worin $R_9$ Wasserstoff, Methyl oder Methoxy ist.

Bevorzugt verwendet man im erfindungsgemäßen Verfahren eine $\beta$-Hydroxypropionsäure der Formel

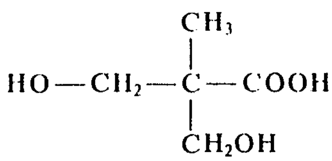 (8)

und Phenylhydrazin zur Herstellung des Hydrazids der Formel

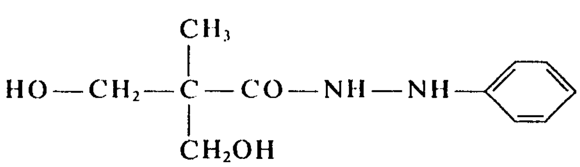 (9)

und überführt dieses in Gegenwart eines sauren Katalysators in das 1-Phenyl-4-methyl-4-hydroxymethyl-pyrazolidin-3-on.

Die Umsetzungen werden in der Regel bei höheren Temperaturen, z. B. 100 bis 160° C durchgeführt. Bevorzugte Temperaturbereiche sind 110 bis 140° C, insbesondere 120 bis 140° C. Besonders geeignet ist der Bereich von 125 bis 135° C. Die Molverhältnisse von $\beta$-Hydroxypropionsäure zur Hydrazinkomponente können so gewählt werden, daß entweder die Säure- oder die Hydrazinkomponente im Überschuß vorliegt. In der Regel wird die Säurekomponente in nicht mehr als 100%igem Überschuß bezogen auf die Hydrazinkomponente verwendet. Dagegen ist es andererseits möglich, mit einem sehr großen Überschuß an Hydrazinkomponente zu arbeiten. Man erhält so z. B. gute Ausbeuten an Pyrazolidin-3-onen, wenn die Hydrazinkomponente als Lösungsmittel eingesetzt wird. Geeignetere Verhältnisse von Säure- zu Hydrazinkomponente liegen jedoch im Bereich von etwa 1 : 5. Interessant sind die Verhältnisse von 1 : 1 bis 1 : 3. Vorzugsweise wird ein Verhältnis von 1 : 2 bis 1 : 3, insbesondere von 1 : 1,5 bis 1 : 2,5 gewählt.

Werden die Komponenten im Molverhältnis 1,5 : 1 bis 2 : 1 eingesetzt, so kann die überschüssige Carbonsäure die Funktion des sauren Katalysators übernehmen, indem sie den Ringschluß des Hydrazids zum 3-Pyrazolidinon bewirkt. Liegen die Ausgangsverbindungen jedoch in den Molverhältnissen 1 : 1 bis 1 : 5 vor, so ist die Gegenwart eines sauren Katalysators für den Ringschluß und für die Bildung des Hydrazids erforderlich. Beispiele für einen solchen Katalysator sind anorganische oder organische Säuren, wie z. B. aliphatische oder aromatische Carbon- oder Sulfonsäuren. Bevorzugt sind Chlor- oder Bromwasserstoffsäure, ortho-Phosphorsäure, Polyphosphorsäure, Phosphorpentoxyd, Pyrophosphorsäure oder ein saures Salz der ortho-Phosphorsäure, ferner Benzolsulfon-, p-Toluolsulfon- oder Methansulfonsäure, Kohlen- oder Schwefeldioxid oder Schwefelsäure. Besonders geeignet sind Chlorwasserstoff, ortho-Phosphorsäure und Methansulfonsäure und Schwefelsäure, Ortho-Phosphorsäure ist ein sehr geeigneter Katalysator.

Es ist auch möglich, im erfindungsgemäßen Verfahren mit einem Gemisch solcher Katalysatoren zu arbeiten. Ferner kann es, da der Katalysator von Anfang an im Reaktionsgemisch anwesend ist, vorteilhaft sein, zu einem bestimmten Zeitpunkt des Verfahrens, z. B. nach Bildung der Hauptmenge an Hydrazid, den ursprünglich verwendeten Katalysator durch einen stärker sauren Katalysator zu ersetzen.

Gemäß einer Variante des erfindungsgemäßen Verfahrens erfolgt die Zyklisierung des Hydrazids durch eine Säure in der Schmelze.

Auch die Bildung des Hydrazids kann durch Zusammenschmelzen von Säure- und Hydrazinkomponente gegebenenfalls in Gegenwart eines sauren Katalysators erreicht werden. Vorzugsweise wird diese Umsetzung jedoch in konzentrierter Lösung durchgeführt. Als Lösungsmittel kommen z. B. Xylol, Toluol, Chlorbenzol, 1,2-Dichlorbenzol und Aethylbenzol in Frage.

Es ist vorteilhaft, das bei der Zyklisierung entstehende Wasser laufend aus dem Reaktionsgemisch zu entfernen. Dafür eignen sich z. B. Lösungsmittel oder Lösungsmittelgemische, die mit Wasser azeotrope Gemische bilden, deren Siedepunkte unterhalb der Reaktionstemperatur liegen. Als Beispiele kommen die oben genannten Lösungsmittel in Frage.

Das Wasser kann auch durch Anlegen von Vakuum aus dem Reaktionsgemisch entfernt werden. Diese Methode eignet sich besonders dann, wenn die Umsetzungen lösungsmittelfrei in der Schmelze durchgeführt werden.

Das erfindungsgemäße Verfahren wird anhand der nachfolgenden Beispiele erläutert.

## A. Herstellung des 1-Phenyl-4-methyl-4-hydroxymethyl-pyrazolidin-3-on

### Beispiel 1

In einem 5 l Vierhalsrundkolben mit Thermometer, Rührer, Einfüllstutzen und Rückflußkühler mit Wasserabscheider werden 3000 ml Xylol vorgelegt. Unter Rühren trägt man 108 g (1,1 Mol) kristallisierte ortho-Phosphorsäure ein, erhitzt das Gemisch mit einem Ölbad auf 100° C und läßt 259,5 g (236 ml, 2,4 Mol) Phenylhydrazin zufließen. Die Temperatur steigt dabei auf 115 bis 120° C, wobei sich eine hellgelbe Suspension bildet. Anschließend werden 80,5 g (0,6 Mol) 2,2-Bishydroxymethylpropionsäure eingetragen. Man erhitzt das Gemisch auf 135° C und trennt das sich bildende Wasser azeotrop im Wasserabscheider ab. Nach 30 Minuten Reaktionszeit fügt man wiederum 80,5 g (0,6 Mol) Bishydroxymethylpropionsäure hinzu und setzt das Erhitzen am Rückfluß während 21 Stunden fort. (Abgeschiedene Menge Wasser ca. 40 ml). Anschließend fügt man dem Reaktionsgemisch 10 g Kieselgur und 4 g Aktivkohle hinzu und filtriert heiß auf einer Porzellannutsche. Aus dem gelben Filtrat kristallisieren nach dem Abkühlen auf 0° C 145 g schwach rosa gefärbtes 1-Phenyl-4-methyl-4-hydroxymethyl-pyrazolidin-3-on aus. Aus dem Rückstand erhält man durch Extraktion mit 500 ml Xylol weitere 9,2 g reines Produkt. Insgesamt ergeben sich 154,2 g (d. h. 62,3% der Theorie) Produkt mit einem Schmelzpunkt von 118 bis 120° C.

Das Dünnschichtchromatogramm (Laufmittel: Chloroform-Methanol = 85 : 15) zeigt die Einheitlichkeit des erhaltenen Produktes (rf 0,5).
Das NMR-Spektrum entspricht der erwarteten Struktur.

Elementaranalyse:

| | | | |
|---|---|---|---|
| berechnet % | C 64,06 | H 6,84 | N 13,58 |
| gefunden % | C 63,51 | H 7,00 | N 13,30 |

Ähnliche gute Resultate erhält man, wenn man als Katalysatoren anstelle von ortho-Phosphorsäure Phosphorpentoxyd, Pyrophosphorsäure oder ein saures Salz der ortho-Phosphorsäure einsetzt.
Ferner erhält man auch gute Ergebnisse, wenn anstelle von insgesamt 2,4 Mol Phenylhydrazin nur 0,6 Mol verwendet werden.

Beispiel 2

In einem 3 l Vierhalsrundkolben mit Thermometer, Rührer, Einfüllstutzen und Rückflußkühler mit Wasserabscheider werden 750 ml Xylol und 1081 g (10 Mol) Phenylhydrazin vorgelegt. Unter Rühren trägt man 223,6 g (1,67 Mol) 2,2-Bishydroxymethylpropionsäure ein und erhitzt das Gemisch mit einem Ölbad zum Rückfluß. Die Temperatur beträgt dann etwa 125 bis 135°C. Das entstehende Wasser wird im Wasserabscheider abgetrennt. Nach 1 Stunde werden weitere 223,6 g Säurekomponente zugegeben und nach einer weiteren Stunde nochmals 223,6 g. Nach etwa 7stündiger Reaktionszeit haben sich 85 ml Wasser gebildet. Anschließend wird das Lösungsmittel bei 150 bis 200 mbar abdestilliert, dann das durch Nebenreaktion gebildete Anilin und überschüssiges Phenylhydrazin bei 20 mbar. Es verbleibt eine Schmelze des Zwischenproduktes 2,2-Dihydroxymethylpropionsäurephenylhydrazid von etwa 125 bis 135°C, die mit 35 g (0,35 Mol) konzentrierter Schwefelsäure versetzt wird. Man hält die Schmelze 3 Stunden bei 130°C und entfernt das sich bildende Wasser durch Anlegen eines Vakuums von 50 bis 60 mbar laufend aus dem Reaktionsgemisch. Nach Aufarbeitung in einem Gemisch aus Wasser und Isopropanol erhält man das 1-Phenyl-4-methyl-4-hydroxymethyl-pyrazolidin-3-on in Ausbeute von 61% (Schmelzpunkt: 114 bis 120°C).

B. Herstellung des 1-Phenyl-4,4-dimethyl-pyrazolidin-3-on

Beispiel 3

Man verfährt wie in Beispiel 6 angegeben, setzt jedoch anstelle von (insgesamt) 1,2 Mol 2,2-Bishydroxymethylpropionsäure 1,2 Mol 2-Methyl-2-hydroxymethylpropionsäure ein. Die Verbindung der Formel

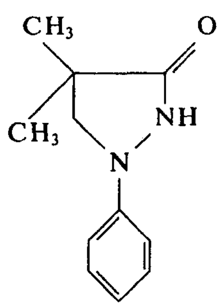

(102)

läßt sich in Ausbeuten von 64,7% isolieren. (Smp.: 132—135°C).

C. Herstellung des 1-p-Tolyl-4,4-dihydroxymethyl-pyrazolidin-3-on

Beispiel 4

Gemäß Beispiel 7 werden 10 Mol p-Tolylhydrazin mit 5 Mol Trishydroxymethylessigsäure umgesetzt. Die Verbindung der Formel

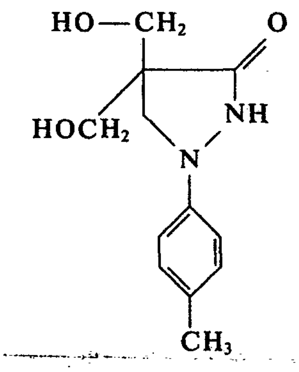

(103)

läßt sich in einer Ausbeute von 58% isolieren. (Smp.: 138—141° C).

**Patentansprüche**

1. Verfahren zur Herstellung von 3-Pyrazolidinonen der Formel

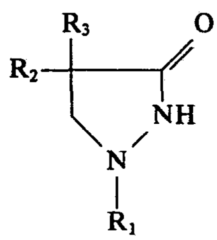

(1)

worin $R_1$ Phenyl oder durch Alkyl oder Alkoxy mit je 1 bis 4 Kohlenstoffatomen, Hydroxyl oder Halogen substituiertes Phenyl, $R_2$ Alkyl oder Hydroxyalkyl mit je 1 bis 4 Kohlenstoffatomen und $R_3$ Hydroxyalkyl mit 1 bis 4 Kohlenstoffatomen ist, durch Umsetzung einer $\beta$-Hydroxypropionsäure der Formel

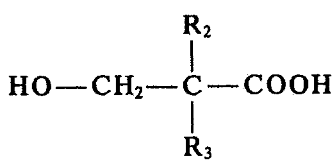

(2)

worin $R_2$ und $R_3$ die oben angegebenen Bedeutungen haben, mit einem Hydrazin der Formel

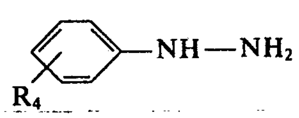

(3)

worin $R_4$ Wasserstoff, Alkyl oder Alkoxy mit je 1 bis 4 Kohlenstoffatomen, Hydroxyl oder Halogen ist, zu einem $\beta$-Hydroxypropionsäurehydrazid und Zyklisierung dieses Hydrazids in Gegenwart einer Säure, dadurch gekennzeichnet, daß man das $\beta$-Hydroxypropionsäurehydrazid in der gleichen Reaktionsmischung zyklisiert, wobei die Bildung des Hydrazids und dessen Zyklisierung in Gegenwart eines sauren Katalysators durchgeführt werden.

2. Verfahren nach Anspruch 1, dadurch gekennzeichnet, daß die Bildung des Hydrazids und die Zyklisierung bei Temperaturen von 100 bis 160° C erfolgen.

3. Verfahren zur Herstellung von 3-Pyrazolidinonen nach einem der Ansprüche 1 bis 2, dadurch gekennzeichnet, daß die Bildung des $\beta$-Hydroxypropionsäurehydrazids in der Schmelze bei 125 bis 135° C erfolgt und man die Zyklisierung dieses Hydrazids in der gleichen Schmelze in Gegenwart eines sauren Katalysators durchführt.

4. Verfahren zur Herstellung von 3-Pyrazolidinonen nach einem der Ansprüche 1 bis 2, dadurch gekennzeichnet, daß man das Hydroxypropionsäurehydrazid in der gleichen Reaktionsmischung in der Schmelze bei 125 bis 135° C in Gegenwart eines sauren Katalysators zyklisiert.

5. Verfahren nach einem der Ansprüche 3 oder 4, dadurch gekennzeichnet, daß man auch die Bildung des Hydrazids in Gegenwart eines sauren Katalysators durchführt.

6. Verfahren nach einem der Ansprüche 1 bis 4, dadurch gekennzeichnet, daß die $\beta$-Hydroxypropionsäure der Formel

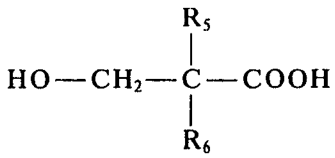

(4)

entspricht, worin $R_5$ Methyl, Aethyl, Hydroxymethyl, 1- oder 2-Hydroxyäthyl und $R_6$ Hydroxymethyl oder 1- oder 2-Hydroxyäthyl ist.

7. Verfahren nach einem der Ansprüche 1 bis 4, dadurch gekennzeichnet, daß das Hydrazin der Formel

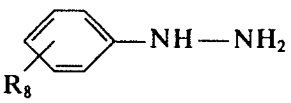

(6)

entspricht, worin $R_8$ Wasserstoff, Methyl, Methoxy, Hydroxyl oder Chlor ist.

8. Verfahren nach einem der Ansprüche 1 bis 4, dadurch gekennzeichnet, daß die Carbonsäure- und Hydrazinkomponente im Molverhältnis 2 : 1 bis 1 : 5 eingesetzt werden.

9. Verfahren nach einem der Ansprüche 1 bis 4, dadurch gekennzeichnet, daß die Zyklisierung des Hydrazids ohne zusätzlichen sauren Katalysator erfolgt, wenn das Molverhältnis von Carbonsäure- zu Hydrazinkomponente 1,5 : 1 bis 2 : 1 beträgt.

10. Verfahren nach einem der Ansprüche 1 bis 4, dadurch gekennzeichnet, daß der saure Katalysator Chlor- oder Bromwasserstoffsäure, ortho-Phosphorsäure, Polyphosphorsäure, Phosphorpentoxyd, Pyrophosphorsäure oder ein saures Salz der ortho-Phosphorsäure, Benzolsulfon-, p-Toluolsulfon- oder Methansulfonsäure, Kohlen- oder Schwefeldioxid oder Schwefelsäure, ist.

11. Verfahren nach Anspruch 1, dadurch gekennzeichnet, daß Bildung und Zyklisierung des Hydrazids in einem Lösungsmittel erfolgen.

**Claims**

1. A process for the preparation of a pyrzolidin-3-one of the formula

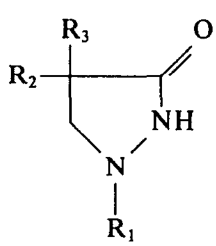

(1)

wherein $R_1$ is phenyl or phenyl substituted by $C_1-C_4$alkyl, $C_1-C_4$alkoxy, hydroxy or halogen, $R_2$ is $C_1-C_4$alkyl or $C_1-C_4$hydroxyalkyl and $R_3$ is $C_1-C_4$hydroxyalkyl, by reacting a $\beta$-hydroxypropionic acid of the formula

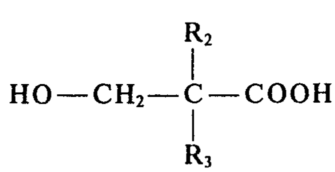

(2)

wherein $R_2$ and $R_3$ are as defined above, with a hydrazine of the formula

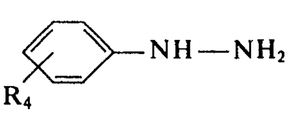

(3)

wherein $R_4$ is hydrogen, $C_1-C_4$alkyl, $C_1-C_4$alkoxy, hydroxy or halogen, to give a $\beta$-hydroxypropionic

acid hydrazide and cyclising said hydrazide in the presence of an acid, which process comprises cyclising the β-hydroxypropionic acid hydrazide in the same reaction mixture, the formation of the hydrazide and the cyclisation thereof optionally being carried out in the presence of an acid catalyst.

2. A process according to claim 1, which comprises carrying out the formation of the hydrazide and the cyclisation in the temperature range from 100° to 160°C.

3. A process for the preparation of a pyrazolidin-3-one according to either claim 1 or claim 2, which process comprises carrying out the formation of the β-hydroxypropionic acid hydrazide in the melt at 125° to 135°C, and carrying out the cyclisation of said hydrazide in the same melt in the presence of an acid catalyst.

4. A process for the preparation of a pyrazolidin-3-one according to either claim 1 or claim 2, which process comprises cyclising the hydroxypropionic acid hydrazide in the same reaction mixture in the melt at 125° to 135°C in the presence of an acid catalyst.

5. A process according to either claim 3 or claim 4, which process comprises also carrying out the formation of the hydrazide in the presence of an acid catalyst.

6. A process according to any one of claims 1 to 4, wherein the β-hydroxypropionic acid is of the formula

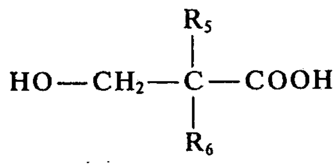

(4)

wherein $R_5$ is methyl, ethyl, hydroxymethyl, 1- or 2-hydroxyethyl and $R_6$ is hydroxymethyl or 1- or 2-hydroxyethyl.

7. A process according to any one of claims 1 to 4, wherein the hydrazine is of the formula

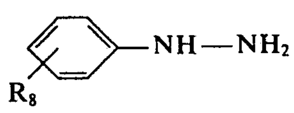

(6)

wherein $R_8$ is hydrogen, methyl, methoxy, hydroxy or chlorine.

8. A process according to any one of claims 1 to 4, wherein the carboxylic acid component and the hydrazine component are employed in a molar ratio of 2 : 1 to 1 : 5.

9. A process according to any one of claims 1 to 4, which comprises carrying out the cyclisation of the hydrazide without additional acid catalyst if the molar ratio of the carboxylic acid component to the hydrazine component is 1,5 : 1 to 2 : 1.

10. A process according to any one of claims 1 to 4, wherein the acid catalyst is hydrochloric acid, hydrobromic acid, orthophosphoric acid, polyphosphoric acid, phosphorus pentoxide, pyrophosphoric acid or an acid salt of orthophosphoric acid, or it is benzenesulfonic acid, p-toluenesulfonic acid, methanesulfonic acid, carbon dioxide, sulfur dioxide or sulfuric acid.

11. A process according to claim 1, which comprises carrying out the formation and cyclisation of the hydrazide in a solvent.

**Revendications**

1. Procédé de préparation de 3-pyrazolidinones de formule:

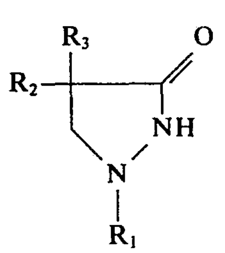

(1)

dans laquelle $R_1$ est un groupe phényle ou un groupe phényle substitué par un radical alkyle ou alcoxy ayant chacun 1 à 4 atomes de carbone, hydroxyle ou halogéno; $R_2$ est un groupe alkyle ou hydroxyalkyle ayant chacun 1 à 4 atomes de carbone, et $R_3$ est un groupe hydroxyalkyle ayant 1 à 4 atomes de carbone, en faisant réagir un acide β-hydroxypropionique de formule:

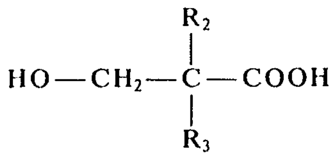

(2)

dans laquelle $R_2$ et $R_3$ ont les significations données ci-dessus, avec une hydrazine de formule:

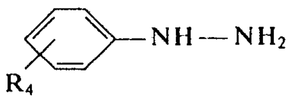

(3)

dans laquelle $R_4$ est un atome d'hydrogène, un groupe alkyle ou alcoxy ayant chacun 1 à 4 atomes de carbone, hydroxyle ou halogéno, pour avoir un β-hydroxypropionyl-hydrazide et en cyclisant cet hydrazide en présence d'un acide, caractérisé par le fait qu'on cyclise le β-hydroxypropionyl-hydrazide dans le même mélange réactionnel, la formation de l'hydrazide et sa cyclisation étant effectuées en présence d'un catalyseur acide.

2. Procédé selon la revendication 1, caractérisé par le fait que la formation de l'hydrazide et la cyclisation sont effectuées à des températures de 100 à 160° C.

3. Procédé de préparation de 3-pyrazolidinones selon l'une des revendications 1 et 2, caractérisé par le fait que la formation du β-hydroxypropionyl-hydrazide s'effectue dans la masse fondue à 125—135° C et qu'on effectue la cyclisation de cet hydrazide dans la même masse fondue en présence d'un catalyseur acide.

4. Procédé de préparation de 3-pyrazolidinones selon l'une des revendications 1 et 2, caractérisé par le fait qu'oun cyclise l'hydroxypropionyl-hydrazide dans le même mélange réactionnel dans la masse fondue à 125—135° C en présence d'un catalyseur acide.

5. Procédé selon l'une des revendications 3 et 4, caractérisé par le fait qu'on effectue également la formation de l'hydrazide en présence d'un catalyseur acide.

6. Procédé selon l'une des revendications 1 à 4, caractérisé par le fait que l'acide β-hydroxypropionique correspond à la formule:

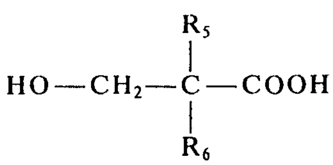

(4)

dans laquelle $R_5$ est un groupe méthyle, éthyle, hydroxyméthyle, 1- ou 2-hydroxyéthyle, et $R_6$ est un groupe hydroxyméthyle ou 1- ou 2-hydroxyéthyle.

7. Procédé selon l'une des revendications 1 à 4, caractérisé par le fait que l'hydrazine correspond à la formule:

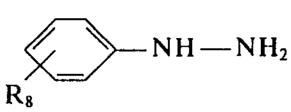

(6)

dans laquelle $R_8$ est un atome d'hydrogène, un groupe méthyle, méthoxy, hydroxyle ou chloro.

8. Procédé selon l'une des revendications 1 à 4, caractérisé par le fait que le constituant acide carboxylique et le constituant hydrazine sont utilisés dans un rapport molaire de 2 : 1 à 1 : 5.

9. Procédé selon l'une des revendications 1 à 4, caractérisé par le fait que la cyclisation de l'hydrazide est effectuée sans ajouter de catalyseur acide quand le rapport molaire du constituant acide carboxylique au constituant hydrazine est de 1,5 : 1 à 2 : 1.

10. Procédé selon l'une des revendications 1 à 4, caractérisé par le fait que le catalyseur acide est l'acide chlorhydrique ou l'acide bromhydrique, l'acide ortho-phosphorique, l'acide polyphosphorique, le pentoxyde de phosphore, l'acide pyrophosphorique ou bien un sel acide de l'acide ortho-phosphorique, l'acide benzènesulfonique, l'acide p-toluènesulfonique ou l'acide méthanesulfonique, le gaz carbonique ou le dioxyde de soufre ou l'acide sulfurique.

11. Procédé selon la revendication 1, caractérisé par le fait que la formation et la cyclisation de l'hydrazide sont effectuées dans un solvant.